(19)

Europäisches Patentamt
European Patent Office
Office européen des brevets

(11) **EP 4 753 251 A1**

(12) **EUROPEAN PATENT APPLICATION**
published in accordance with Art. 153(4) EPC

(43) Date of publication:
**03.06.2026 Bulletin 2026/23**

(21) Application number: **24859848.4**

(22) Date of filing: **28.08.2024**

(51) International Patent Classification (IPC):
**H04N 1/46** (2006.01)  **G06T 1/00** (2006.01)

(52) Cooperative Patent Classification (CPC):
**A61B 3/06; G06T 1/00; H04N 1/46; H04N 1/60**

(86) International application number:
**PCT/JP2024/030783**

(87) International publication number:
**WO 2025/047818 (06.03.2025 Gazette 2025/10)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **30.08.2023 PCT/JP2023/031584**

(71) Applicant: **Resonac Corporation
Tokyo 105-7325 (JP)**

(72) Inventors:
• **SAKAGUCHI, Suguru
Tokyo 105-7325 (JP)**
• **TOMITA, Miyuki
Tokyo 105-7325 (JP)**

(74) Representative: **Strehl & Partner mbB
Maximilianstrasse 54
80538 München (DE)**

(54) **COLOR GENERATION SYSTEM, COLOR GENERATION PROGRAM, METHOD FOR PRODUCING DYE OR LIGHT EMITTER, DYE, AND LIGHT EMITTER**

(57) A color generation system includes: an acquisition unit configured to acquire a reference spectrum which is a spectrum of a reference color and a subject model indicating a color vision system of a subject; a response calculation unit configured to calculate, as a reference response, a response from a color vision system of a reference observer who has visually recognized the reference color, based on a predetermined reference model indicating the color vision system of the reference observer and the reference spectrum; a spectrum calculation unit configured to calculate a conversion spectrum for causing the subject model to output a response that matches or approximates the reference response; and a generation unit configured to generate a corresponding spectrum which is a spectrum of a corresponding color corresponding to the reference color, based on the conversion spectrum.

*Fig.1*

EP 4 753 251 A1

## Description

## Technical Field

An aspect of the present disclosure relates to a color generation system, a color generation program, a method for producing a colorant or a light emitter, a colorant, and a light emitter.

## Background Art

Color processing in consideration of persons with color vision deficiency has been known in the related art. For example, a color processing device described in Patent Literature 1 acquires a color value to be subjected to color processing, calculates a color vision degree coefficient representing a degree of color vision deficiency based on a distribution of the number of persons in examination results obtained by a color vision examination conducted on a predetermined group, calculates a color conversion coefficient used for converting the acquired color value based on a correspondence between the color vision degree coefficient and sensitivity characteristics of L cones, M cones, and S cones, and performs color conversion processing on the color value using the color conversion coefficient.

## Citation List

## Patent Literature

[Patent Literature 1] Japanese Patent No. 5924289

## Summary of Invention

## Technical Problem

There is a demand for a mechanism for providing colors in consideration of color vision diversity.

## Solution to Problem

A color generation system according to an aspect of the present disclosure includes: an acquisition unit configured to acquire a reference spectrum which is a spectrum of a reference color and a subject model indicating a color vision system of a subject; a response calculation unit configured to calculate, as a reference response, a response from a color vision system of a reference observer who has visually recognized the reference color, based on a predetermined reference model indicating the color vision system of the reference observer and the reference spectrum; a spectrum calculation unit configured to calculate a conversion spectrum for causing the subject model to output a response that matches or approximates the reference response; and a generation unit configured to generate a corresponding spectrum which is a spectrum of a corresponding color corresponding to the reference color, based on the conversion spectrum.

A color generation program according to an aspect of the present disclosure causes a computer to execute: acquiring a reference spectrum which is a spectrum of a reference color and a subject model indicating a color vision system of a subject; calculating, as a reference response, a response from a color vision system of a reference observer who has visually recognized the reference color, based on a predetermined reference model indicating the color vision system of the reference observer and the reference spectrum; calculating a conversion spectrum for causing the subject model to output a response that matches or approximates the reference response; and generating a corresponding spectrum which is a spectrum of a corresponding color corresponding to the reference color, based on the conversion spectrum.

In these aspects, a conversion spectrum capable of causing the subject to perceive the reference color in the same manner as the reference observer is calculated, and a spectrum of a corresponding color corresponding to the reference color (corresponding spectrum) is generated based on the conversion spectrum. The corresponding spectrum makes it possible to provide colors in consideration of color vision diversity.

A method for producing a colorant or a light emitter according to an aspect of the present disclosure includes: acquiring a reference spectrum which is a spectrum of a reference color and a subject model indicating a color vision system of a subject; calculating, as a reference response, a response from a color vision system of a reference observer who has visually recognized the reference color, based on a predetermined reference model indicating the color vision system of the reference observer and the reference spectrum; calculating a conversion spectrum for causing the subject model to output a response that matches or approximates the reference response; generating a corresponding spectrum which is a spectrum of a corresponding color corresponding to the reference color, based on the conversion spectrum; and producing a colorant or a light emitter of the corresponding color based on the corresponding spectrum.

In this aspect, a conversion spectrum capable of causing the subject to perceive the reference color in the same manner as the reference observer is calculated, and a spectrum of a corresponding color corresponding to the reference color (corresponding spectrum) is generated based on the conversion spectrum. Then, a colorant or a light emitter of the corresponding color is produced based on the corresponding spectrum. By using the colorant or the light emitter, it is possible to provide colors in consideration of color vision diversity.

A colorant according to an aspect of the present disclosure has a corresponding spectrum based on a reference spectrum which is a spectrum of a reference color, wherein a reflectance indicated by the corresponding spectrum is 0.025 or less in a range of 540 to 560 nm, and wherein the corresponding spectrum includes a region in which the reflectance is 0.2 or more in each of a range of 380 to 540 nm and a range of 560 to 730 nm.

By using a colorant having such characteristics, it is possible to provide colors in consideration of color vision diversity.

A light emitter according to an aspect of the present disclosure has a corresponding spectrum based on a reference spectrum which is a spectrum of a reference color, wherein a ratio of a maximum intensity of the corresponding spectrum in a range of 380 to 540 nm to a maximum intensity of the corresponding spectrum in a range of 540 to 560 nm is 8.0 or more, and wherein a ratio of a maximum intensity of the corresponding spectrum in a range of 560 to 730 nm to the maximum intensity of the corresponding spectrum in the range of 540 to 560 nm is 8.0 or more.

By using a light emitter having such characteristics, it is possible to provide colors in consideration of color vision diversity.

**Advantageous Effects of Invention**

According to an aspect of the present disclosure, it is possible to provide colors in consideration of color vision diversity.

**Brief Description of Drawings**

FIG. 1 is a diagram showing an example of a functional configuration of a color generation system.
FIG. 2 is a graph showing an example of sensitivity characteristics of L cones, M cones, and S cones.
FIG. 3 is a flowchart showing an example of an operation of the color generation system.
FIG. 4 is a diagram showing a relationship between a reference spectrum and a corresponding spectrum.
FIG. 5 is a diagram showing a relationship between a reference spectrum and a corresponding spectrum.
FIG. 6 is a diagram showing a relationship between a reference spectrum and a corresponding spectrum.
FIG. 7 is a diagram showing a relationship between a reference spectrum and a corresponding spectrum.
FIG. 8 is a diagram showing another example of a functional configuration of a color generation system.
FIG. 9 is a flowchart showing another example of an operation of the color generation system.

**Description of Embodiments**

Hereinafter, embodiments of the present disclosure will be described in detail with reference to the accompanying drawings. In the description of the drawings, the same or equivalent elements are denoted by the same reference numerals, and a redundant description thereof will be omitted.

[Outline of System]

A color generation system according to the present disclosure is a computer system or a control system for providing colors in consideration of color vision diversity. Color vision diversity refers to a situation in which recognition or discrimination of colors differs among people. The color generation system may be used for the purpose of facilitating a common recognition of colors among people having different color visions or promoting mutual understanding regarding recognition or discrimination of colors.

As types of color vision, there are five types: C-type (Common), P-type (Protanope), D-type (Deuteranope), T-type (Tritanope), and A-type (Achromatic).

The C-type is a group having three types of cones: L cones, M cones, and S cones. The L cones are photoreceptor cells that respond with high sensitivity to light near long wavelengths such as red. The M cones are photoreceptor cells that respond with high sensitivity to light near medium wavelengths such as green. The S cones are photoreceptor cells that respond with high sensitivity to light near short wavelengths such as blue. In the case of Japanese people, 95% of males and 99% of females belong to the C-type. People of the C-type are also referred to as persons with normal color vision.

On the other hand, people of the P-type, D-type, T-type, and A-type are also referred to as persons with color vision deficiency. The P-type is a group consisting of protanopia in which L cones are absent and protanomaly in which the sensitivity of L cones is shifted to resemble the sensitivity of M cones. The D-type is a group consisting of deuteranopia in which M cones are absent and deuteranomaly in which the sensitivity of M cones is shifted to resemble the sensitivity of L cones. The T-type is a group in which S cones are absent. The A-type is a group having only one type of cone or having no cones at all. Among persons with color vision deficiency, the P-type and D-type account for the majority, and the proportions of the T-type and A-type are extremely small.

As an example of promoting the above-described common recognition or mutual understanding, the color generation system according to the present disclosure may be used to allow a person with color vision deficiency to experience a color recognized by a person with normal color vision or to allow a person with normal color vision to experience a color recognized by a person with color vision deficiency. Alternatively, the color generation system may be used to provide a color that is recognized in the same manner by both a person with normal color vision and a person with color vision deficiency. The color generation system may be used for the purpose of promoting common recognition or mutual understanding of colors among people of the C-type, P-type, D-type, T-type, and A-type.

The color generation system generates a corresponding color, which is another color in consideration of color vision diversity, based on a reference color. The reference color is a color designated for generating the corresponding color. The corresponding color may be said to be a color corresponding to the reference color. In one example, the color generation system generates a corresponding spectrum, which is a spectrum of the corresponding color, based on a reference spectrum which is a spectrum of the reference color. In another example, the color generation system produces a colorant or a light emitter of the corresponding color based on the corresponding spectrum.

[Configuration of System]

The color generation system is constituted by one or more computers. In a case where a plurality of computers are used, these computers are connected via a communication network such as the Internet or an intranet, to logically construct one color generation system.

A computer constituting the color generation system generally includes, as hardware devices, a processor, a memory, a communication interface, an input device, and an output device. Examples of the processor include a CPU and a GPU. The memory may be constituted by a flash memory, a hard disk, and the like. The communication interface may be constituted by a network card or a wireless communication module. Examples of the input device include a keyboard, a pointing device, a touch panel, a microphone, a sensor, and a camera. Examples of the output device include a monitor, a touch panel, a head-mounted display (HMD), and a speaker.

A color generation program for causing a computer to function as the color generation system includes program code for implementing each functional module of the color generation system. The color generation program may be recorded on a tangible recording medium such as a CD-ROM, a DVD-ROM, or a semiconductor memory in a non-transitory manner and then provided. Alternatively, the color generation program may be provided as data signals superimposed on carrier waves via a communication network. The provided color generation program is stored in, for example, the memory.

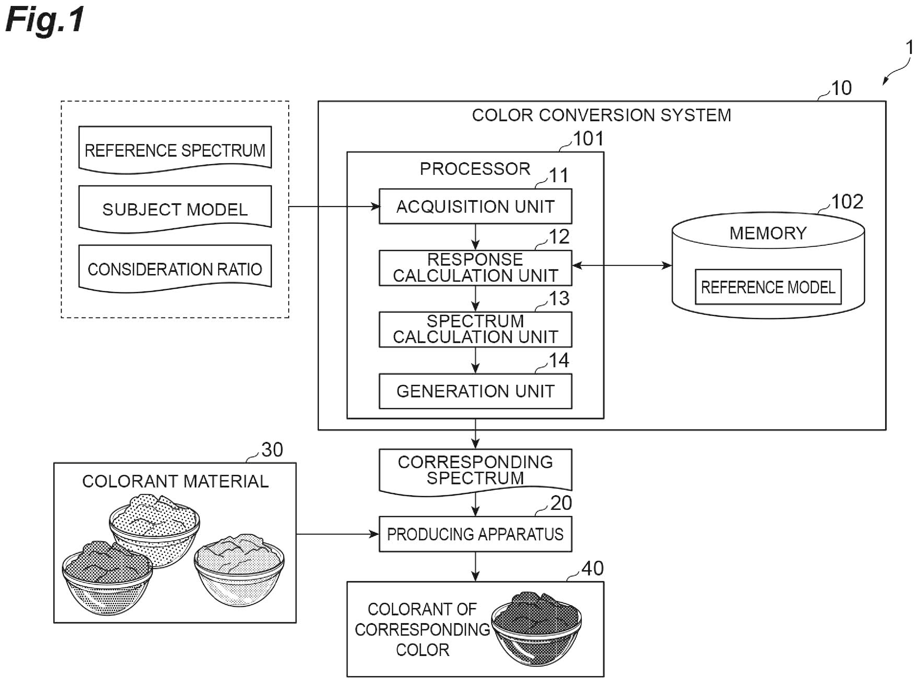

A configuration of a color generation system 1 according to one example will be described with reference to FIG. 1. FIG. 1 is a diagram showing a functional configuration of the color generation system 1.

The color generation system 1 includes a color conversion system 10. The color conversion system 10 is a computer system that generates a corresponding spectrum based on a reference spectrum. The color conversion system 10 is constituted by one computer or a plurality of computers connected to each other via a communication network. The color conversion system 10 includes a processor 101 and a memory 102. In one example, the processor 101 functions as an acquisition unit 11, a response calculation unit 12, a spectrum calculation unit 13, and a generation unit 14. The memory 102 stores in advance a predetermined reference model which is data indicating a color vision system of a reference observer. At least one of a person with normal color vision and a person with color vision deficiency is assumed as the reference observer. The reference model may be prepared for each of the person with normal color vision and the person with color vision deficiency. In a case where a person with color vision deficiency is assumed as the reference observer, the reference model may be prepared for each type of color vision, and for example, a reference model for the P-type, a reference model for the D-type, a reference model for the T-type, and a reference model for the A-type may be prepared. Furthermore, a reference model in which a difference in degree such as strong or weak color vision deficiency is considered may be prepared.

The acquisition unit 11 is a functional module that acquires input data indicating a reference spectrum, a subject model, and a consideration ratio. The subject model is data indicating a color vision system of a subject. The subject refers to a person assumed as a target to whom the corresponding color is provided. The subject may be a person with normal color vision or may be a person with color vision deficiency of a specific type such as the P-type or the D-type. The consideration ratio is an index indicating a degree of change from the reference color to the corresponding color.

The response calculation unit 12 is a functional module that calculates, as a reference response, a response from a color vision system of a person who has visually recognized the reference color. The response calculation unit 12 calculates the reference response based on the reference spectrum and the reference model. The response corresponds to electrical signals transmitted from the L cones, M cones, and S cones to the brain through the optic nerve.

The spectrum calculation unit 13 is a functional module that calculates a conversion spectrum for causing the subject model to output a response that matches or approximates the reference response. The "response that matches or approximates the reference response" refers to a response in which a difference from the reference response is equal to or less than a predetermined threshold value.

The generation unit 14 is a functional module that generates a corresponding spectrum based on at least the conversion spectrum. In one example, the generation unit 14 generates the corresponding spectrum based on the reference spectrum, the conversion spectrum, and the consideration ratio.

The color generation system 1 may further include a producing apparatus 20. The producing apparatus 20 is an apparatus or a group of apparatuses that produces a colorant 40 of the corresponding color from one or more colorant materials 30 prepared in advance, based on the corresponding spectrum. The colorant refers to a substance that imparts color to an object. The colorant may be a dye or a pigment.

[Color Vision Model]

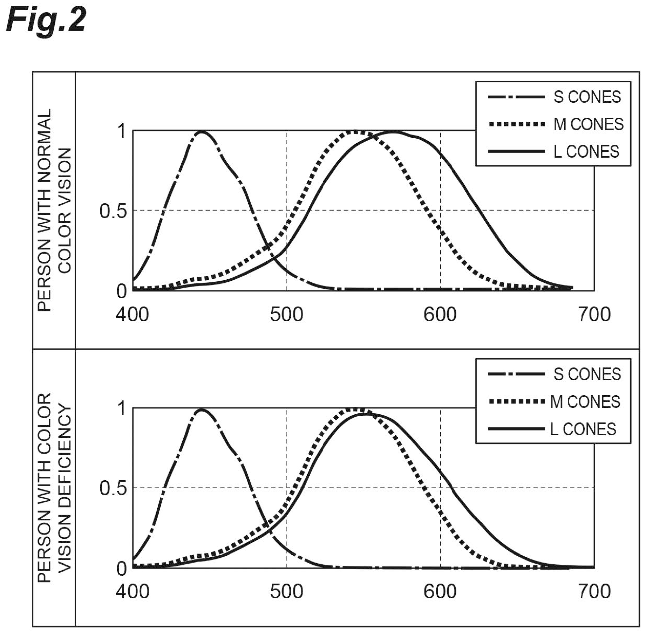

Both the reference model and the subject model may be said to be color vision models indicating a color vision system of a person. In one example, the color vision model indicates sensitivity characteristics of each of L cones, M cones, and S cones. The sensitivity characteristics are also referred to as spectral characteristics. FIG. 2 is a graph showing an example of sensitivity characteristics of each cone for a person with normal color vision and a person with color vision deficiency. The horizontal axis and the vertical axis of the graph indicate wavelength (nm) and relative sensitivity, respectively. The person with color vision deficiency shown in FIG. 2 corresponds to protanomaly. The person with color vision deficiency has a greater degree of overlap between the sensitivity characteristics of M cones and L cones compared to a person with normal color vision (C-type). Such a difference in sensitivity characteristics causes a difference in recognition or discrimination of colors.

[Operation of System]

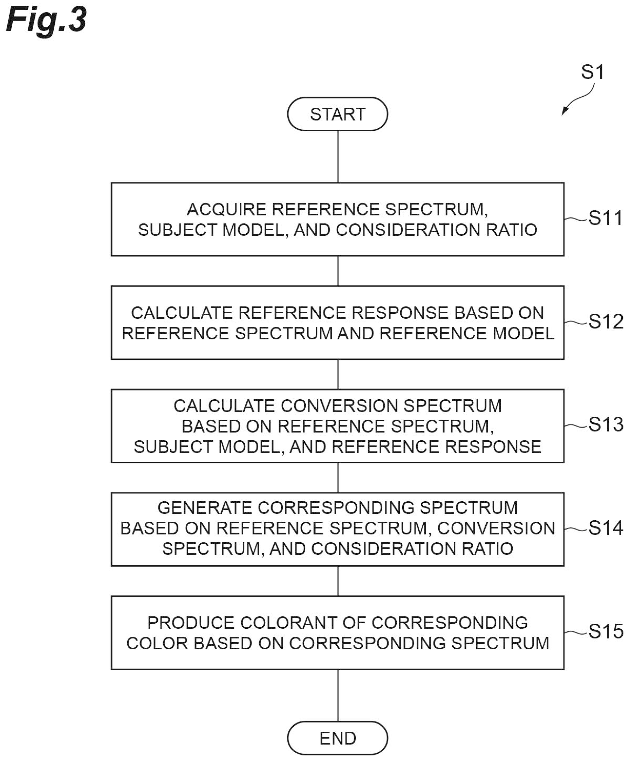

A color generation method executed by the color generation system 1 will be described with reference to FIG. 3. FIG. 3 is a flowchart showing an example of an operation of the color generation system 1 as a processing flow S1.

In step S11, the acquisition unit 11 acquires a reference spectrum, a subject model, and a consideration ratio. In one example, a user of the color conversion system 10 inputs a reference color, a type of color vision of the subject, and a consideration ratio. The acquisition unit 11 receives the input and reads a reference spectrum corresponding to the reference color and a subject model corresponding to the type of color vision from a predetermined storage device. Alternatively, the acquisition unit 11 may receive input of the reference spectrum, the subject model, and the consideration ratio. Alternatively, the acquisition unit 11 may receive these data from another computer.

In one example, the reference spectrum is represented by reflectance (%) at each wavelength (nm) in the visible light region. The reference spectrum may be expressed by a spectrum function indicating a relationship between wavelength and reflectance.

In one example, the subject model indicates sensitivity characteristics of each of L cones, M cones, and S cones as shown in FIG. 2. The subject model may be expressed by a sensitivity function indicating a relationship between wavelength and relative sensitivity. The sensitivity function may be prepared for each of L cones, M cones, and S cones. As described above, the subject model indicates a color vision system (color vision model) of either a person with normal color vision or a person with color vision deficiency. The subject model may be a color vision model obtained by measuring the sensitivity of L cones, M cones, and S cones of an individual (subject), or may be a color vision model based on representative values for protanomaly, deuteranomaly, or the like.

In one example, the consideration ratio is represented by a numerical value greater than 0% and equal to or less than 100%. In a case where the consideration ratio is 0%, the corresponding color is the same as the reference color. In a case where the consideration ratio is 100%, a color recognized by the subject who has viewed the corresponding color matches or approximates a color recognized by the reference observer who has viewed the reference color. That is, in a case where the consideration ratio is 100%, a situation in which the reference observer views the reference color may be experienced by the subject through the corresponding color. In a case where the consideration ratio is a specific numerical value or within a numerical value range, colors recognized by both the subject and the reference observer who have viewed the corresponding color may match or substantially match. Such a consideration ratio may be, for example, 20% or a value close thereto.

In step S12, the response calculation unit 12 calculates a reference response based on the reference spectrum and the reference model. The reference response may be defined by a response $\alpha$ from L cones, a response $\beta$ from M cones, and a response $\gamma$ from S cones, and in the present disclosure, the reference response is expressed as $(\alpha, \beta, \gamma)$.

In one example, the response calculation unit 12 calculates, for each of L cones, M cones, and S cones of the reference observer, a response from the cone that has sensed the reference color as follows. The response calculation unit 12 calculates a product of the relative sensitivity of the cone and the reflectance indicated by the reference spectrum at each wavelength within the visible light region. The product may be said to indicate a degree of excitation. The response calculation unit 12 obtains a sum of the products over the respective wavelengths as the response from the cone.

Assuming that the wavelength is represented by x, the sensitivity functions of L cones, M cones, and S cones are represented by $f_L(x)$, $f_M(x)$, and $f_S(x)$, respectively, and the spectrum function of the reference color is represented by $g(x)$, the responses $\alpha$, $\beta$, and $\gamma$ may be expressed by Equations (1) to (3), respectively.

$$\alpha = \int f_L(x)g(x)dx \ \dots \ (1)$$

$$\beta = \int f_M(x)g(x)dx \ \dots \ (2)$$

$$\gamma = \int f_S(x)g(x)dx \ ... \ (3)$$

That is, the response calculation unit 12 may calculate, as the reference response $(\alpha, \beta, \gamma)$, an integral of a product of the sensitivity function and the spectrum function for each cone.

In step S13, the spectrum calculation unit 13 calculates a conversion spectrum based on the reference spectrum, the subject model, and the reference response. In one example, the spectrum calculation unit 13 changes at least a part of the reference spectrum to generate a candidate spectrum. Then, the spectrum calculation unit 13 inputs the candidate spectrum to the subject model and calculates, as a subject response $(\alpha', \beta', \gamma')$, a response from a color vision system of the subject who has visually recognized a color indicated by the candidate spectrum. The spectrum calculation unit 13 determines whether the subject response $(\alpha', \beta', \gamma')$ matches or approximates the reference response $(\alpha, \beta, \gamma)$. The spectrum calculation unit 13 searches for a subject response $(\alpha', \beta', \gamma')$ that matches or approximates the reference response $(\alpha, \beta, \gamma)$ while changing at least a part of the reference spectrum, and finally identifies the conversion spectrum. The conversion spectrum may also be said to be the corresponding spectrum in a case where the consideration ratio is 100%.

In step S14, the generation unit 14 generates a corresponding spectrum based on the reference spectrum, the conversion spectrum, and the consideration ratio. In one example, the generation unit 14 combines the reference spectrum and the conversion spectrum by linear combination based on the consideration ratio to generate the corresponding spectrum. Assuming that the reference spectrum, the conversion spectrum, and the consideration ratio are represented by $S_d$, $S_v$, and r, respectively, the corresponding spectrum $S_c$ is expressed by Equation (4). r is greater than 0 and equal to or less than 1. In a case where r = 1, that is, the consideration ratio is 100%, the generation unit 14 sets the conversion spectrum as the corresponding spectrum without modification.

$$S_c = (1 - r) \cdot S_d + r \cdot S_v \ ... \ (4)$$

In step S15, the producing apparatus 20 produces the colorant 40 of the corresponding color based on the generated corresponding spectrum. In one example, the producing apparatus 20 refers to the corresponding spectrum, selects one or more colorant materials 30 from a plurality of colorant materials 30 prepared in advance, and determines a mixing ratio for each of the selected colorant materials 30. Then, the producing apparatus 20 mixes the selected one or more colorant materials 30 at the determined mixing ratio to produce the colorant 40. The producing apparatus 20 may generate the colorant 40 according to one or more colorant materials 30 and a mixing ratio that are selected by an operator. In any case, the colorant 40 has a corresponding spectrum based on the reference spectrum and contributes to color expression in consideration of color vision diversity.

[Colorant]

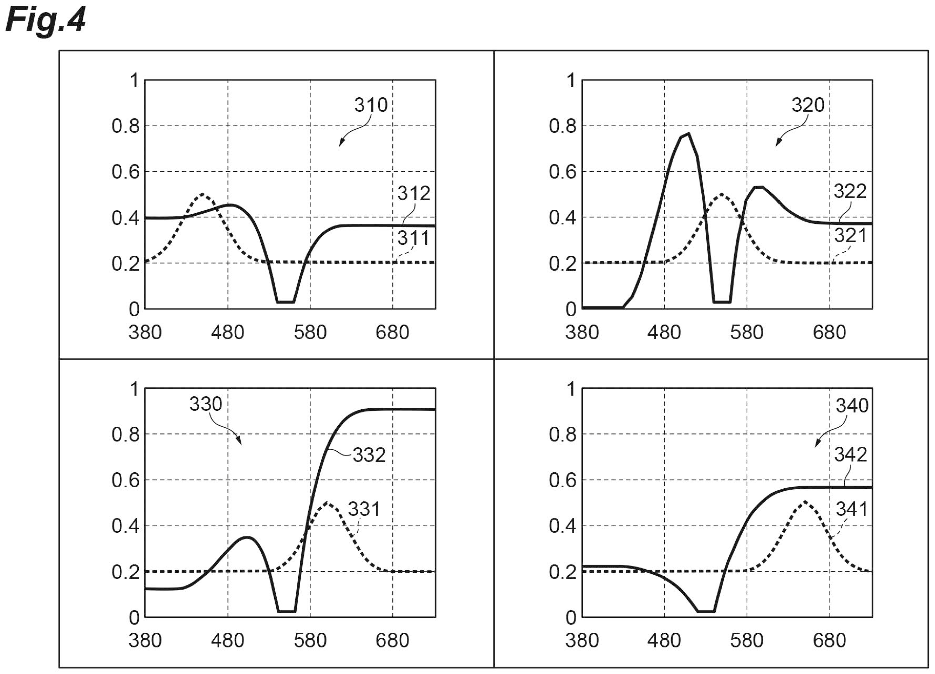

A configuration of the colorant 40 produced by the color generation system 1 (producing apparatus 20) will be described with reference to FIGS. 4 to 7. Each of FIGS. 4 to 7 is a diagram showing a relationship between a reference spectrum and a corresponding spectrum in a graph. In each graph, the horizontal axis and the vertical axis indicate wavelength (nm) and reflectance (%), respectively.

FIG. 4 shows four examples 310, 320, 330, and 340. Each of these examples shows a pair of a Gaussian function-type reference spectrum and a corresponding spectrum generated for a person of the P-type based on the reference spectrum. The example 310 shows a reference spectrum 311 and a corresponding spectrum 312 generated based on the reference spectrum 311 with a consideration ratio of 100%. Corresponding spectra 322, 332, and 342 correspond to reference spectra 321, 331, and 341, respectively. The consideration ratio is also 100% for the corresponding spectra 322, 332, and 342.

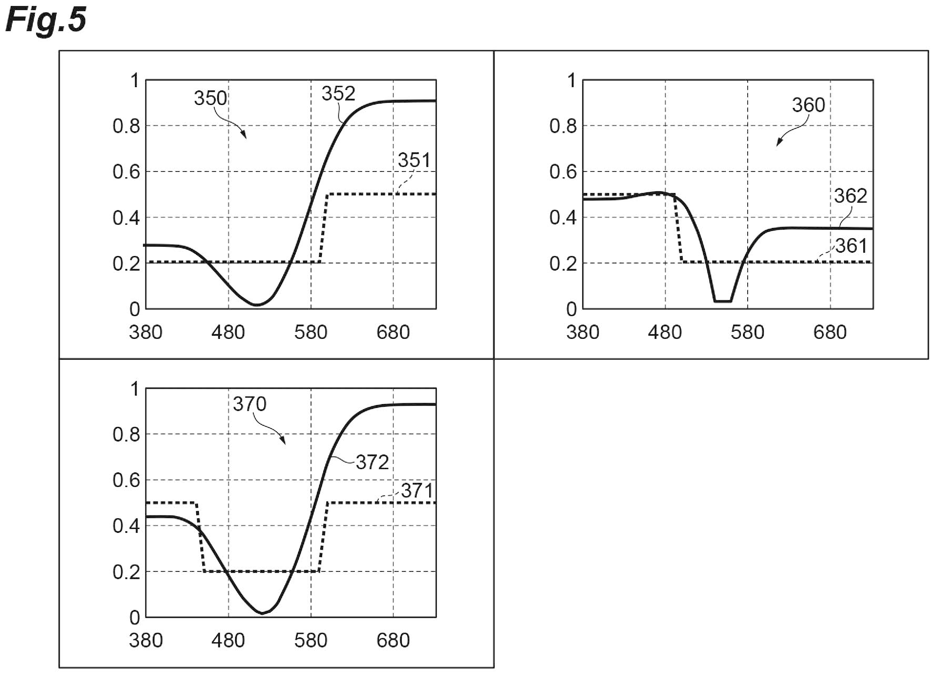

FIG. 5 shows three examples 350, 360, and 370. Each of these examples shows a pair of a step function-type reference spectrum and a corresponding spectrum generated for a person of the P-type based on the reference spectrum. Corresponding spectra 352, 362, and 372 correspond to reference spectra 351, 361, and 371, respectively. The consideration ratio is also 100% for the corresponding spectra 352, 362, and 372.

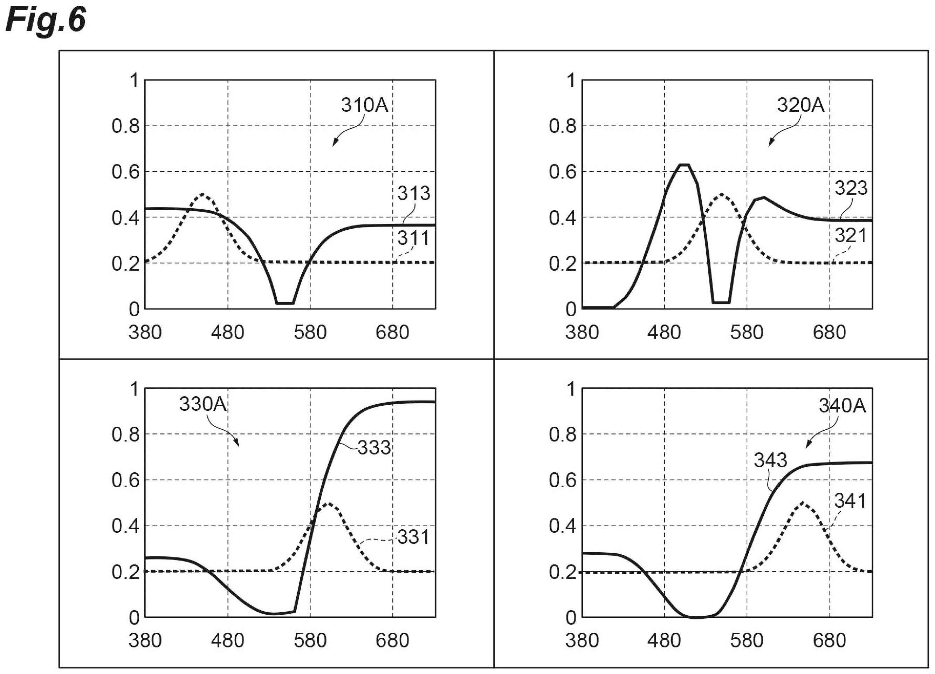

FIG. 6 shows four examples 310A, 320A, 330A, and 340A. Each of these examples shows a pair of a Gaussian function-type reference spectrum and a corresponding spectrum generated for a person of the D-type based on the reference spectrum. Corresponding spectra 313, 323, 333, and 343 correspond to reference spectra 311, 321, 331, and 341, respectively. The consideration ratio is 100% for each of the corresponding spectra 313, 323, 333, and 343.

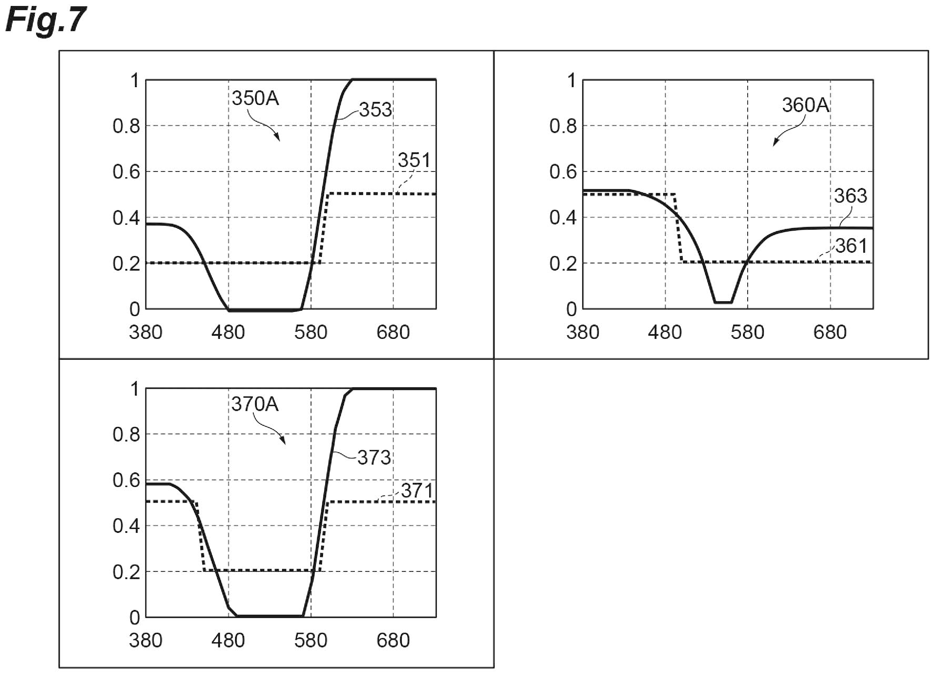

FIG. 7 shows three examples 350A, 360A, and 370A. Each of these examples shows a pair of a step function-type reference spectrum and a corresponding spectrum generated for a person of the D-type based on the reference spectrum. Corresponding spectra 353, 363, and 373 correspond to reference spectra 351, 361, and 371, respectively. The consideration ratio is also 100% for the corresponding spectra 353, 363, and 373.

As shown in FIGS. 4 to 7, the corresponding spectrum and the colorant 40 obtained by the color generation system 1 may have the following two characteristics.

- The reflectance indicated by the corresponding spectrum is 0.025 or less in the range of 540 to 560 nm.
- The corresponding spectrum includes a region in which the reflectance is 0.2 or more in each of the range of 380 to 540 nm and the range of 560 to 730 nm.

The colorant 40 is a result of substantially not using the range of 540 to 560 nm, in which the degree of overlap between L cones and M cones tends to be large in the P-type and D-type, and of actively utilizing the reflectance in the long wavelength region that is mainly absorbed by L cones. The colorant 40 makes it possible to allow a person with color vision deficiency to experience a situation in which a person with normal color vision recognizes the reference color, or to realize the reverse thereof.

The colorant 40 may be used to produce various articles. For example, the colorant 40 may be used for paints or inks, may be used for stationery such as writing instruments, sticky notes, and notebooks, or may be used for publications such as picture books, paintings, and photo books. Alternatively, the colorant 40 may be used for other articles such as clothes, fabrics, furniture, accessories, and signboards. Through articles using the colorant 40, it is possible to facilitate a common recognition of colors among persons with normal color vision and persons with color vision deficiency or to promote mutual understanding regarding recognition or discrimination of colors. It is also possible to provide persons with color vision deficiency with an opportunity to enjoy using articles in the same manner as persons with normal color vision. Alternatively, through articles using the colorant 40, it may also be expected to give persons with color vision deficiency confidence in color selection or to reduce mistakes in color selection.

[Light Emitter and Producing Thereof]

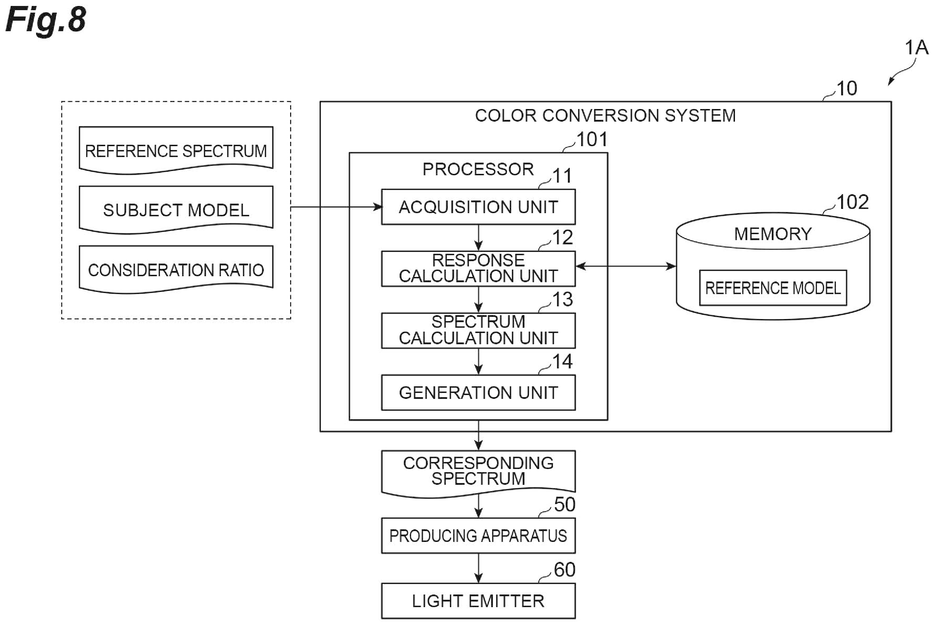

A configuration of a color generation system 1A according to one example will be described with reference to FIG. 8. FIG. 8 is a diagram showing a functional configuration of the color generation system 1A. The color generation system 1A includes the above-described color conversion system 10 and a producing apparatus 50. The producing apparatus 50 is an apparatus or a group of apparatuses that produces a light emitter 60 of the corresponding color based on the corresponding spectrum. The light emitter refers to an article that emits light by excitation energy (electrical energy, chemical energy, light energy, and the like). As shown in the example of FIG. 8, the color conversion system 10 may be used not only for producing a colorant but also for producing a light emitter.

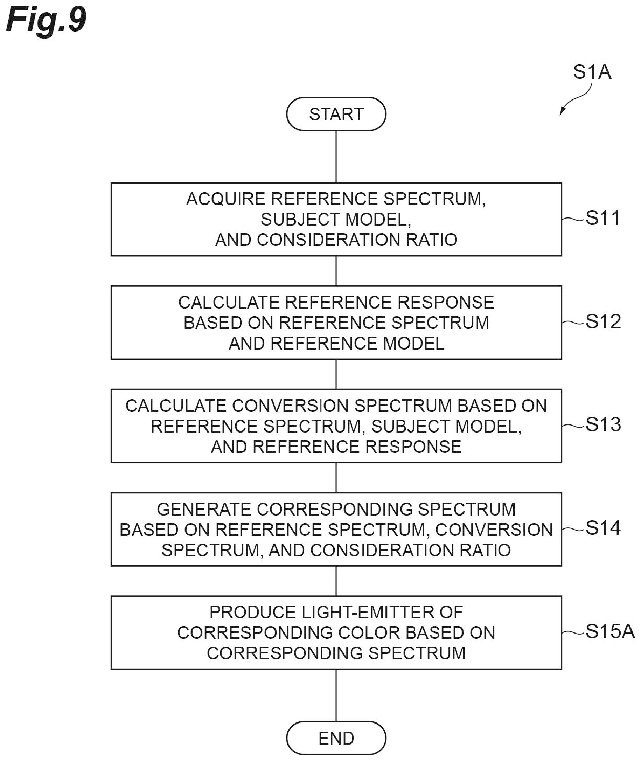

A color generation method executed by the color generation system 1A will be described with reference to FIG. 9. FIG. 9 is a flowchart showing an example of an operation of the color generation system 1A as a processing flow S1A. The processing flow S1A differs from the above-described processing flow S1 in that it includes step S15A instead of the above-described step S15. Hereinafter, the step S15A will be described.

In step S15A, the producing apparatus 50 produces the light emitter 60 of the corresponding color based on the generated corresponding spectrum. In one example, the producing apparatus 50 refers to the corresponding spectrum, selects one or more colors from a plurality of colors prepared in advance, and determines a mixing ratio for phosphors of each selected color. Then, the producing apparatus 50 produces the light emitter 60 having one or more types of phosphors corresponding to the selected one or more colors at the determined mixing ratio. Phosphors of each color prepared for producing the light emitter 60 may be produced by, for example, a method disclosed as Example 1 in Japanese Unexamined Patent Publication No. 2009-161372. Quantum dots (quantum dot phosphors) disclosed in this document have a sharp emission spectrum with a full width at half maximum (FWHM) of about 30 nm. The producing apparatus 50 may generate the light emitter 60 according to one or more colors (that is, one or more types of phosphors) and a mixing ratio selected by an operator. In any case, the light emitter 60 has a corresponding spectrum based on the reference spectrum and contributes to color expression in consideration of color vision diversity.

Similar to the above-described colorant 40, the light emitter 60 produced by the color generation system 1A (producing apparatus 50) also shows a relationship between a reference spectrum and a corresponding spectrum as shown in FIGS. 4 to 7. In the case of the light emitter 60, the vertical axis of each graph in FIGS. 4 to 7 indicates relative intensity. As shown in FIGS. 4 to 7, the corresponding spectrum and the light emitter 60 obtained by the color generation system 1A may have the following two characteristics.

- A ratio of a maximum intensity Mb of the corresponding spectrum in the range of 380 to 540 nm to a maximum intensity Ma of the corresponding spectrum in the range of 540 to 560 nm is 8.0 or more. That is, $Mb/Ma \geq 8.0$.
- A ratio of a maximum intensity Mc of the corresponding spectrum in the range of 560 to 730 nm to the maximum intensity Ma of the corresponding spectrum in the range of 540 to 560 nm is 8.0 or more. That is, $Mc/Ma \geq 8.0$.

The light emitter 60 is a result of substantially not using the range of 540 to 560 nm, in which the degree of overlap between L cones and M cones tends to be large in the P-type and D-type, and actively utilizing the intensity in the long wavelength region that is mainly absorbed by L cones. The light emitter 60 makes it possible to allow a person with color vision deficiency to experience a situation in which a person with normal color vision recognizes the reference color, or to realize the reverse thereof.

The light emitter 60 may be used to produce various articles. The light emitter 60 may be a phosphor or a phosphorescent material. The light emitter 60 may be used for, for example, displays, traffic lights, lighting, wavelength conversion films, backlights, indicators, luminescent dyes, luminescent paints, or luminescent inks. Through articles using the light emitter 60, it is possible to facilitate a common recognition of colors among persons with normal color vision and persons with color vision deficiency or to promote mutual understanding regarding recognition or discrimination of colors. It is also possible to provide persons with color vision deficiency with an opportunity to enjoy using articles in the same manner as persons with normal color vision. Alternatively, through articles using the light emitter 60, it may also be expected to give persons with color vision deficiency confidence in color selection or to reduce mistakes in color selection.

[Modification Examples]

The technology according to the present disclosure has been described in detail above based on various examples. However, the present disclosure is not limited to the above examples. The technology according to the present disclosure may be modified in various ways without departing from the gist of the present disclosure.

In the above examples, the color conversion system 10 generates the corresponding spectrum based on the consideration ratio. However, in a case where the color generation system (conversion system) sets the conversion spectrum as the corresponding spectrum without modification, that is, in a case where the corresponding spectrum with a consideration ratio of 100% is generated, the color generation system (conversion system) does not need to acquire and use the consideration ratio.

A producing apparatus that produces a colorant or a light emitter of the corresponding color may be provided in a computer system or a control system separate from the color generation system.

A processing procedure of a method executed by at least one processor is not limited to the above examples. For example, some of the above-described steps may be omitted, or the steps may be executed in a different order. In addition, any two or more of the above-described steps may be combined, or some of the steps may be corrected or deleted. Alternatively, other steps may be executed in addition to each of the above-described steps.

In the comparison between the magnitudes of two numerical values in the present disclosure, either of two criteria of "equal to or greater than" and "greater than" may be used, or either of two criteria of "equal to or less than" and "less than" may be used.

In the present disclosure, an expression "At least one processor executes a first process, executes a second process, ... and executes an n-th process." or an expression corresponding thereto indicates a concept including a case where an entity that executes n processes from the first process to the n-th process, that is, a processor, is changed in the middle. That is, this expression indicates a concept including both a case where all of the n processes are executed by the same processor and a case where the processor is changed according to any policy in the n processes.

Articles in which the colorant or the light emitter according to the present disclosure is used are classified into various categories. The categories include, for example, food, clothing, and housing, clothing/fashion, school/children, brand/advertising, toys, and others. Examples of specific articles included in these categories are as follows. Articles related to food, clothing, and housing are, for example, clothes, foods containing colorants, tableware, tablecloths, wallpaper, furniture, bedding, and the like. Articles related to food, clothing, and housing may be a combination of wallpaper, furniture, bedding, and the like with lighting. Articles related to clothing/fashion are, for example, umbrellas, hats, hairpins, hair ties, headbands, hair bands, glasses, sunglasses, clip-on earrings, pierced earrings, earphones, cosmetics, articles related to nail art (nail polish, gel, stickers, and the like), necklaces, neckties, scarves, winter scarves, shawls, tie pins, innerwear tops, outerwear tops, innerwear bottoms, outerwear bottoms, gloves, wristbands, bracelets, watches, smartwatches, rings, smartphone cases, bags, belts, socks, leggings, tights, stockings, anklets, shoes, sandals, geta, slippers, fans, folding fans, handkerchiefs, towels, and the like. Articles related to school/children are, for example, stationery, notebooks, letter sets, toolboxes, school bags, textbooks/teaching materials, uniforms, gym clothes, indoor shoes, paint sets, lunch boxes, crayons, sticky notes, files, binders, and the like. Articles related to brand/advertising are, for example, articles with logos, signboards, articles related to advertising, packages (beverages, confectionery, book covers, CD jackets), and the like. Articles related to toys are, for example, stuffed animals/dolls, playhouse items, clay, and the like. Other articles are, for example, candles, soaps, business cards, and the like. As described above, articles according to the present disclosure are diverse, and specific articles are included in each category. This makes it possible to accommodate various uses or scenes.

[Appendix]

As will be appreciated from the various examples above, the present disclosure includes the following aspects.

(Appendix 1)

A color generation system comprising:

an acquisition unit configured to acquire a reference spectrum which is a spectrum of a reference color and a subject model indicating a color vision system of a subject;

a response calculation unit configured to calculate, as a reference response, a response from a color vision system of a reference observer who has visually recognized the reference color, based on a predetermined reference model indicating the color vision system of the reference observer and the reference spectrum;

a spectrum calculation unit configured to calculate a conversion spectrum for causing the subject model to output a response that matches or approximates the reference response; and

a generation unit configured to generate a corresponding spectrum which is a spectrum of a corresponding color corresponding to the reference color, based on the conversion spectrum.

(Appendix 2)

The color generation system according to Appendix 1,

wherein the acquisition unit is further configured to acquire a consideration ratio indicating a degree of change from the reference color to the corresponding color, and

wherein the generation unit is configured to generate the corresponding spectrum based on the reference spectrum, the conversion spectrum, and the consideration ratio.

(Appendix 3)

The color generation system according to Appendix 2, wherein the generation unit is configured to combine the reference spectrum and the conversion spectrum by linear combination based on the consideration ratio to generate the corresponding spectrum.

(Appendix 4)

The color generation system according to Appendix 1, wherein the generation unit is configured to set the conversion spectrum as the corresponding spectrum.

(Appendix 5)

The color generation system according to any one of Appendices 1 to 4,

wherein the subject model indicates sensitivity characteristics of each of L cones, M cones, and S cones of the subject,

wherein the reference model indicates sensitivity characteristics of each of L cones, M cones, and S cones of the reference observer,

wherein the response calculation unit is configured to calculate a response from each of L cones, M cones, and S cones of the reference observer as the reference response, and

wherein the spectrum calculation unit is configured to calculate the conversion spectrum based on a response from each of L cones, M cones, and S cones of the subject.

(Appendix 6)

A color generation program causing a computer to execute:

acquiring a reference spectrum which is a spectrum of a reference color and a subject model indicating a color vision system of a subject;

calculating, as a reference response, a response from a color vision system of a reference observer who has visually recognized the reference color, based on a predetermined reference model indicating the color vision system of the reference observer and the reference spectrum;

calculating a conversion spectrum for causing the subject model to output a response that matches or approximates the reference response; and

generating a corresponding spectrum which is a spectrum of a corresponding color corresponding to the reference color, based on the conversion spectrum.

(Appendix 7)

A method for producing a colorant or a light emitter, the method comprising:

acquiring a reference spectrum which is a spectrum of a reference color and a subject model indicating a color vision system of a subject;

calculating, as a reference response, a response from a color vision system of a reference observer who has visually recognized the reference color, based on a predetermined reference model indicating the color vision system of the reference observer and the reference spectrum;

calculating a conversion spectrum for causing the subject model to output a response that matches or approximates the reference response;

generating a corresponding spectrum which is a spectrum of a corresponding color corresponding to the reference color, based on the conversion spectrum; and

producing a colorant or a light emitter of the corresponding color based on the corresponding spectrum.

(Appendix 8)

A colorant having a corresponding spectrum based on a reference spectrum which is a spectrum of a reference color,

wherein a reflectance indicated by the corresponding spectrum is 0.025 or less in a range of 540 to 560 nm, and
wherein the corresponding spectrum includes a region in which the reflectance is 0.2 or more in each of a range of 380 to 540 nm and a range of 560 to 730 nm.

(Appendix 9)

A light emitter having a corresponding spectrum based on a reference spectrum which is a spectrum of a reference color,

wherein a ratio of a maximum intensity of the corresponding spectrum in a range of 380 to 540 nm to a maximum intensity of the corresponding spectrum in a range of 540 to 560 nm is 8.0 or more, and
wherein a ratio of a maximum intensity of the corresponding spectrum in a range of 560 to 730 nm to the maximum intensity of the corresponding spectrum in the range of 540 to 560 nm is 8.0 or more.

According to Appendices 1 and 6, a conversion spectrum capable of causing the subject to perceive the reference color in the same manner as the reference observer is calculated, and a spectrum of a corresponding color corresponding to the reference color (corresponding spectrum) is generated based on the conversion spectrum. The corresponding spectrum makes it possible to provide colors in consideration of color vision diversity.

According to Appendix 2, by using the consideration ratio, a desired corresponding spectrum may be precisely generated. For example, a spectrum of a color that is recognized in the same manner by both a person with normal color vision and a person with color vision deficiency may be generated.

According to Appendix 3, since the reference spectrum and the conversion spectrum are combined by linear combination, the corresponding spectrum may be generated by simple calculation.

According to Appendix 4, since the conversion spectrum is set as the corresponding spectrum without modification, it is possible to allow a person with color vision deficiency to experience a color recognized by a person with normal color vision, or to realize the reverse thereof.

According to Appendix 5, since the subject model and the reference model that simulate an actual color vision system of a human are used, the corresponding spectrum may be generated more accurately.

According to Appendix 7, a conversion spectrum capable of causing the subject to perceive the reference color in the same manner as the reference observer is calculated, and a spectrum of a corresponding color corresponding to the reference color (corresponding spectrum) is generated based on the conversion spectrum. Then, a colorant or a light emitter of the corresponding color is produced based on the corresponding spectrum. By using the colorant or the light emitter, it is possible to provide colors in consideration of color vision diversity.

According to Appendix 8, by using a colorant having the above characteristics, it is possible to provide colors in consideration of color vision diversity.

According to Appendix 9, by using a light emitter having the above characteristics, it is possible to provide colors in consideration of color vision diversity.

**Reference Signs List**

1, 1A: Color generation system; 10: Color conversion system; 11: Acquisition unit; 12: Response calculation unit; 13: Spectrum calculation unit; 14: Generation unit; 20: Producing apparatus; 30: Colorant material; 40: Colorant; 50: Producing apparatus; 60: Light emitter.

**Claims**

1. A color generation system comprising:

an acquisition unit configured to acquire a reference spectrum which is a spectrum of a reference color and a subject model indicating a color vision system of a subject;
a response calculation unit configured to calculate, as a reference response, a response from a color vision system of a reference observer who has visually recognized the reference color, based on a predetermined reference model indicating the color vision system of the reference observer and the reference spectrum;
a spectrum calculation unit configured to calculate a conversion spectrum for causing the subject model to output a response that matches or approximates the reference response; and
a generation unit configured to generate a corresponding spectrum which is a spectrum of a corresponding color corresponding to the reference color, based on the conversion spectrum.

2. The color generation system according to claim 1,

wherein the acquisition unit is further configured to acquire a consideration ratio indicating a degree of change from the reference color to the corresponding color, and
wherein the generation unit is configured to generate the corresponding spectrum based on the reference spectrum, the conversion spectrum, and the consideration ratio.

3. The color generation system according to claim 2, wherein the generation unit is configured to combine the reference spectrum and the conversion spectrum by linear combination based on the consideration ratio to generate the corresponding spectrum.

4. The color generation system according to claim 1, wherein the generation unit is configured to set the conversion spectrum as the corresponding spectrum.

5. The color generation system according to any one of claims 1 to 4,

 wherein the subject model indicates sensitivity characteristics of each of L cones, M cones, and S cones of the subject,
 wherein the reference model indicates sensitivity characteristics of each of L cones, M cones, and S cones of the reference observer,
 wherein the response calculation unit is configured to calculate a response from each of L cones, M cones, and S cones of the reference observer as the reference response, and
 wherein the spectrum calculation unit is configured to calculate the conversion spectrum based on a response from each of L cones, M cones, and S cones of the subject.

6. A color generation program causing a computer to execute:

 acquiring a reference spectrum which is a spectrum of a reference color and a subject model indicating a color vision system of a subject;
 calculating, as a reference response, a response from a color vision system of a reference observer who has visually recognized the reference color, based on a predetermined reference model indicating the color vision system of the reference observer and the reference spectrum;
 calculating a conversion spectrum for causing the subject model to output a response that matches or approximates the reference response; and
 generating a corresponding spectrum which is a spectrum of a corresponding color corresponding to the reference color, based on the conversion spectrum.

7. A method for producing a colorant or a light emitter, the method comprising:

 acquiring a reference spectrum which is a spectrum of a reference color and a subject model indicating a color vision system of a subject;
calculating, as a reference response, a response from a color vision system of a reference observer who has visually recognized the reference color, based on a predetermined reference model indicating the color vision system of the reference observer and the reference spectrum;
calculating a conversion spectrum for causing the subject model to output a response that matches or approximates the reference response;
generating a corresponding spectrum which is a spectrum of a corresponding color corresponding to the reference color, based on the conversion spectrum; and
producing a colorant or a light emitter of the corresponding color based on the corresponding spectrum.

8. A colorant having a corresponding spectrum based on a reference spectrum which is a spectrum of a reference color,

 wherein a reflectance indicated by the corresponding spectrum is 0.025 or less in a range of 540 to 560 nm, and
 wherein the corresponding spectrum includes a region in which the reflectance is 0.2 or more in each of a range of 380 to 540 nm and a range of 560 to 730 nm.

9. A light emitter having a corresponding spectrum based on a reference spectrum which is a spectrum of a reference color,

 wherein a ratio of a maximum intensity of the corresponding spectrum in a range of 380 to 540 nm to a maximum intensity of the corresponding spectrum in a range of 540 to 560 nm is 8.0 or more, and
 wherein a ratio of a maximum intensity of the corresponding spectrum in a range of 560 to 730 nm to the maximum intensity of the corresponding spectrum in the range of 540 to 560 nm is 8.0 or more.

**Fig.1**

*Fig.2*

# Fig.3

S1

START

ACQUIRE REFERENCE SPECTRUM, SUBJECT MODEL, AND CONSIDERATION RATIO —S11

CALCULATE REFERENCE RESPONSE BASED ON REFERENCE SPECTRUM AND REFERENCE MODEL —S12

CALCULATE CONVERSION SPECTRUM BASED ON REFERENCE SPECTRUM, SUBJECT MODEL, AND REFERENCE RESPONSE —S13

GENERATE CORRESPONDING SPECTRUM BASED ON REFERENCE SPECTRUM, CONVERSION SPECTRUM, AND CONSIDERATION RATIO —S14

PRODUCE COLORANT OF CORRESPONDING COLOR BASED ON CORRESPONDING SPECTRUM —S15

END

Fig.4

EP 4 753 251 A1

Fig.5

Fig.6

*Fig.7*

360A 363 361

350A 353 351

370A 373 371

**Fig.8**

# Fig.9

S1A

START

ACQUIRE REFERENCE SPECTRUM,
SUBJECT MODEL,
AND CONSIDERATION RATIO —S11

CALCULATE REFERENCE RESPONSE
BASED ON REFERENCE SPECTRUM
AND REFERENCE MODEL —S12

CALCULATE CONVERSION SPECTRUM BASED ON
REFERENCE SPECTRUM, SUBJECT MODEL,
AND REFERENCE RESPONSE —S13

GENERATE CORRESPONDING SPECTRUM
BASED ON REFERENCE SPECTRUM, CONVERSION
SPECTRUM, AND CONSIDERATION RATIO —S14

PRODUCE LIGHT-EMITTER OF
CORRESPONDING COLOR BASED ON
CORRESPONDING SPECTRUM —S15A

END

## INTERNATIONAL SEARCH REPORT

International application No.

**PCT/JP2024/030783**

### A. CLASSIFICATION OF SUBJECT MATTER

*H04N 1/46*(2006.01)i; *G06T 1/00*(2006.01)i

FI:    H04N1/46; G06T1/00 510

According to International Patent Classification (IPC) or to both national classification and IPC

### B. FIELDS SEARCHED

Minimum documentation searched (classification system followed by classification symbols)

H04N1/46; G06T1/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2014-160391 A (FUJI XEROX CO., LTD.) 04 September 2014 (2014-09-04) fig. 1-7 | 1-9 |
| A | JP 2021-12316 A (AISIN SEIKI CO., LTD.) 04 February 2021 (2021-02-04) fig. 2-6 | 1-9 |
| A | JP 2019-88383 A (AISIN SEIKI CO., LTD.) 13 June 2019 (2019-06-13) fig. 1 | 1-9 |
| A | JP 10-83445 A (BUNKYO DAIGAKU GAKUEN) 31 March 1998 (1998-03-31) entire text, all drawings | 1-9 |

☐ Further documents are listed in the continuation of Box C.  ☑ See patent family annex.

| | | |
|---|---|---|
| * | Special categories of cited documents: | |
| "A" | document defining the general state of the art which is not considered to be of particular relevance | |
| "D" | document cited by the applicant in the international application | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **15 October 2024** | **29 October 2024** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (July 2022)

**INTERNATIONAL SEARCH REPORT**
Information on patent family members

International application No.

**PCT/JP2024/030783**

| Patent document cited in search report | | | Publication date (day/month/year) | Patent family member(s) | | | Publication date (day/month/year) |
|---|---|---|---|---|---|---|---|
| JP | 2014-160391 | A | 04 September 2014 | US | 2014/0233070 | A1 | |
| | | | | fig. 1-7 | | | |
| JP | 2021-12316 | A | 04 February 2021 | (Family: none) | | | |
| JP | 2019-88383 | A | 13 June 2019 | (Family: none) | | | |
| JP | 10-83445 | A | 31 March 1998 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5924289 B
- JP 2009161372 A